# EUROPÄISCHE PATENTSCHRIFT

(11) **EP 0 938 513 B1**
(45) Veröffentlichungstag und Bekanntmachung des Hinweises auf die Patenterteilung: **28.03.2001**
(21) Anmeldenummer: 97950104.6
(22) Anmeldetag: 05.11.1997
(51) Int. Cl.: C08G 18/70, C08G 18/24, C07C 263/18

(54) **ZINNHALTIGE POLYISOCYANATE UND VERFAHREN ZUR HERSTELLUNG VON POLYISOCYANAT-POLYADDITIONSPRODUKTEN**
POLYISOCYANATES CONTAINING TIN AND METHOD FOR PREPARING POLYISOCYANATE-POLYADDITION PRODUCTS
POLYISOCYANATES CONTENANT DE L'ETAIN ET PROCEDE POUR PREPARER DES PRODUITS DE POLYADDITION DE POLYISOCYANATE

(30) Priorität: 15.11.1996 DE 19647296
(43) Veröffentlichungstag der Anmeldung: 01.09.1999
(73) Patentinhaber: BASF AKTIENGESELLSCHAFT, 67056 Ludwigshafen (DE)
(72) Erfinder: REICHELT, Michael, D-01945 Ruhland (DE); HEINZ, Marion, D-02994 Bernsdorf (DE); ROTERMUND, Udo, D-01990 Ortrand (DE); HEMPEL, Renate, D-01945 Ruhland (DE); HENSIEK, Rainer, D-49328 Melle (DE); SEIFERT, Holger, D-49448 Hüde (DE)
(86) Internationale Anmeldenummer: EP9706114
(87) Internationale Veröffentlichungsnummer: WO9822520

(56) Entgegenhaltungen:
- EP-A- 0 203 874
- EP-A- 0 409 199
- EP-A- 0 561 568
- GB-A- 2 068 986
- US-A- 5 340 852

## Beschreibung

Die Erfindung betrifft die Verwendung von Polyisocyanaten, enthaltend Zinn in Form von Verbindungen des vier- oder/und zweiwertigen Zinns in einer Menge von 0,1 bis 20 ppm Zinn, bezogen auf die Masse der Polyisocyanate, zur Herstellung von Hartschaumstoffen auf der Basis von Polyurethanen und/oder Polyisocyanuraten mit einer verringerten Rißbildung.

Ferner betrifft die Erfindung ein Verfahren zur Herstellung von Hartschaumstoffen mit einer verringerten Rißbildung auf der Basis von Polyurethanen und/oder Polyisocyanuraten.

Die Herstellung von Hartschaumstoffen auf Isocyanatbasis durch Umsetzung von Polyisocyanaten mit gegenüber Isocyanaten reaktiven Substanzen sowie gegebenenfalls Hilfs- und Zusatzstoffen ist bekannt ("Kunststoff-Handbuch", Band 7, Polyurethane, 3. Auflage, 1993, herausgegeben von G. Oertel, Carl-Hanser-Verlag, München). Neben den Polyurethanen (PUR) entstehen, besonders bei Verwendung entsprechender Katalysatoren, auch Polyisocyanurate (PIR) durch Reaktion der Isocyanate miteinander.

Verwendung finden die Hartschaumstoffe, die üblicherweise auf der Basis von Diphenylmethandiisocyanat (MDI) und/oder Polyphenyl-polymethylen-polyisocyanaten verschiedener Kondensationsstufen (PMDI) kontinuierlich, z.B. mittels eines Doppel-Transportbandes, oder in Formwerkzeugen hergestellt werden, vorzugsweise als Wärmeisolationsmaterial, beispielsweise in Kühlschränken, als Isolationsplatten, als Rohrisolierungen oder als Sandwichelemente.

Die Herstellungsparameter und Eigenschaften der Hartschaumstoffe auf Isocyanatbasis hängen vor allem von der Rezeptur, aber auch von der Temperaturführung und der Schaumstoff dichte ab. Zur Beschleunigung des Produktionsprozesses werden üblicherweise bekannte Katalysatoren wie z.B. tertiäre Amine, Eisen- oder Zinnsalze zur Beschleunigung der Polyurethanbildung oder z.B. Kaliumacetat für die Bildung der PIR eingesetzt. Die Katalyse ist nicht beliebig zu beschleunigen, da eine ausreichend lange Fließfähigkeit des Reaktionsgemisches für das vollständige Ausfüllen von Formwerkzeugen gegeben sein muß. Des weiteren neigen besonders Schaumstoffe mit einer Schichtdicke von mehr als 25 cm, insbesondere Blockschäume, die eine Dicke von mehr 1 m aufweisen können, u.a. durch den starken Temperaturanstieg zu einer verstärkten Rißbildung bei einer zu schnellen Reaktion des Gemisches, insbesonders bei Katalyse mit Eisen-(III)-chlorid. Die Schwierigkeit, einen reproduzierbaren optimalen Reaktionsablauf zu erreichen, wird erschwert durch die Instabilität mancher Katalysatoren sowie der Schwerlöslichkeit von z.B. Eisen-(III)-chlorid in der Isocyanatkomponente.

Für die Katalyse der Reaktion PUR-Bildung aus Polyisocyanaten und Polyhydroxylverbindungen sind Konzentrationen an Dimethylzinnmercaptid von 200 bis 10000 ppm als Katalysator in der Isocyanatkomponente beschrieben (US 4 386 166). Bekanntlich können zinnorganische Verbindungen, besonders mit Mercaptogruppen, Redoxreaktionen (Costa, G., Gazz. Chim. Ital., 89, 42-62, 1950) und somit einer schnellen Alterung unterliegen, so daß eine Lagerstabilität dieser Mischungen nicht gewährleistet ist. Katalysatorgehalte von 500 bis 3000 ppm Dibutylzinndiacetat oder Zinn-IV-octoat bzw. 100 bis 300 ppm Dibutylzinndilaurat in der Isocyanatkomponente sind für die Polyisocyanat-Polyadditionsreaktion beschrieben (JP-A-5 8145 736), allerdings sind diese sehr hohen Konzentrationen für die Formverschäumung oder die kontinuierliche PUR-Herstellung mittels des Doppel-Transportband-Verfahrens aufgrund der extremen Tendenz zur Rißbildung nicht einsetzbar.

Eine verlängerte Lagerfähigkeit der Katalysatoren bei einem Gehalt von 100 ppm in dem Reaktionsgemisch auf Basis aliphatischer Isocyanate kann insbesondere durch Vermeidung von Ketonen im Gemisch und durch Komplexierung der empfindlichen Katalysatoren erreicht werden (WO 95/29007). Dieses Verfahren, bei dem auf Wasser verzichtet wird, ist allerdings für die Herstellung von Hartschäumen nicht anwendbar, weil deren Herstellung auf Wasser als Treibmittel und üblicherweise auf aromatischen und nicht den wesentlich weniger reaktiven und lagerstabileren aliphatischen Isocyanaten beruht. Eine Zugabe der Katalysatoren zu der Komponente, die die gegenüber den Isocyanaten reaktiven Verbindungen enthält, ist aufgrund des üblichen Wassergehaltes von ≥0,05 Gew.-% zur Initiierung der Schaumbildung nicht möglich, da sich die Katalysatoren insbesondere bei einer längeren Lagerung in der Komponente zersetzen.

EP-A-0 561 568 offenbart eine reaktive Zusammensetzung aus Polyisocyanat, die 30 bis 500 ppm eines metallorganischen Katalysators zur Urethanbildung, bevorzugt einer Zinnverbindung, und 30 bis 10 000 ppm einer chlorierten Acylverbindung enthält und Lagerfähigkeit aufweist.

EP-A-0 409 199 offenbart Polyurethan-Polyisocyanurat-Hartschäume, hergestellt aus Polyisocyanaten, Polyolen, Katalysatoren zur Trimerisation und einem Treibmittel. Als Katalysatoren für die Trimerisation können Zinnverbindungen eingesetzt werden.

Der Erfindung lag somit die Aufgabe zugrunde, lagerfähige Polyisocyanate, die Zinnkatalysatoren enthalten, zu entwickeln, die für die Herstellung von PUR- oder PUR/PIR-Hartschaumstoffen, besonders solchen mit einer großen Dicke, geeignet sind.

Diese Aufgabe konnte erfindungsgemäß durch die Verwendung von Polyisocyanaten, enthaltend Zinn in Form von Verbindungen des vier- oder/und zweiwertigen Zinns in einer Menge von 0,1 bis 20 ppm Zinn, bezogen auf die Masse der Polyisocyanate, zur Herstellung von Hartschaumstoffen auf der Basis von Polyurethanen und/ oder Polyisocyanuraten mit einer verringerten Rißbildung gelöst werden. Bevorzugt verwendet werden Verbindungen des vier- oder/ und zweiwertigen Zinns der folgenden Formel für das vierwertige Zinn: RₙSnX₍₄₋ₙ₎ bzw. der folgenden Formel für das zweiwertige Zinn: RₘSnX₍₂₋ₘ₎, in denen R einen aliphatischen Rest, einen substituierten oder unsubstituierten aromatischen Rest, einen Carbonsäurerest, einen Carbonyl-, Mercaptid- und/oder Thiocarbonylrest mit 1 bis 30 Kohlenstoffen bedeutet, X einen anorganischen Rest darstellt, n 0, 1, 2, 3 oder 4 und m 0, 1 oder 2 bedeuten. Besonders bevorzugt sind organische Reste R mit 5 bis 11 Kohlenstoffatomen. Als anorganischer Rest X können z.B. Halogenide genannt werden. Besonders bevorzugt verwendet man als Zinnverbindungen Dibutylzinndilaurat und Di-n-octylzinnmercaptide sowie Salze des zweiwertigen Zinns mit Carbonsäuren, insbesondere Zinn(II)-octoat, das Salz der 2-Ethylhexansäure mit Zinn(II). Die besonders bevorzugten Zinnverbindungen weisen zudem den Vorteil auf, daß sie bei Raumtemperatur flüssig sind und sich gut in das Polyisocyanat einmischen lassen.

Die Polyisocyanate können zusätzlich zu den erfindungsgemäßen Zinnverbindungen weitere übliche Zusätze wie beispielsweise übliche Treibmittel zur Herstellung von Polyisocyanat-Polyadditionsprodukten enthalten. Solche Polyisocyanatmischungen zur Herstellung von Polyisocyanat-Polyadditionsprodukten werden üblicherweise auch Polyisocyanatkomponenten genannt.

Bevorzugt setzt man die Zinnverbindungen in einer Menge von 1 bis 15 ppm Zinn, besonders bevorzugt 2 bis 10 ppm Zinn, bezogen auf die Masse der Polyisocyanate, ein. Es ist überraschend, daß diese Zinnmengen zu lagerstabilen Isocyanatkomponenten führen und des weiteren bei der Umsetzung zu Hartschäumen die Rißbildung deutlich vermindern.

Als Polyisocyanate können die allgemein üblichen aromatischen, aliphatische oder/und cycloaliphatischen Diisocyanate verwendet werden. Beispiele für die aromatischen Diisocyanate sind: 1,5-Naphtylen-Diisocyanate (1,5-NDI), 2,4- und 2,6-Toluylendiisocyanat (TDI) sowie deren Gemische, 2,4'-, 2,2'-, und vorzugsweise 4,4'-Diphenylmethan-diisocyanat (MDI) sowie Mischungen aus mindestens zwei dieser Isomere, Mischungen aus 4,4'- und 2,4'-Diphenylmethan-diisocyanat, Polyphenyl-polymethylen-polyisocyanate, Mischungen aus 2,4'-, 2,2'-, und 4,4'-Diphenylmethandiisocyanaten und Polyphenyl-polymethylen-polyisocyanaten (Roh-MDI), Mischungen aus Roh-MDI und Toluylen-Diisocyanaten, 3,3'-Dimethyldiphenyl-diisocyanat, z.B. 3,3'-Dimethyl-4,4'-diisocyanatdiphenyl, 1,2-Diphenylethan-diisocyanat und Phenyldiisocyanat, vorzugsweise 1,4-Phenyldiisocyanat (PPDI). Die aromatischen Isocyanate können einzeln oder im Gemisch von mindestens zwei verschiedenen Isocyanaten eingesetzt werden. Als aliphatische Polyisocyanate können verzweigtkettige oder vorzugsweise lineare Diisocyanate, beispielsweise mit 4 bis 12 Kohlenstoffatomen, vorzugsweise 4 bis 6 Kohlenstoffatomen, verwendet werden. Hier seien im einzelnen genannt: 1,12-Dodecan-, 2-Ethyl-1,4-butan-, 2-Methyl-1,5-pentan- oder/und 1,4-Butandiisocyanat, bevorzugt 1,6-Hexamethylen-diisocyanat (HDI). Als cycloaliphatische Diisocyanate kommen beispielsweise solche mit 6 bis 18 Kohlenstoffatomen, vorzugsweise 6 bis 12 Kohlenstoffatomen, im alkylsubstituierten oder nicht-alkylsubstituierten Cycloalkylrest in Betracht, z.B.: Cyclohexan-1,3- oder/und -1,4-diisocyanat, 2,4- oder/und 2,6-Hexahydro-toluylen-diisocyanat, 4,4'-, 2,4'-, oder/und 2,2' Dicyclohexylmethan-diisocyanat, vorzugsweise 1-Isocyanato-3,3,5,-trimethyl-5-isocyanato-methylcyclohexan (IPDI). Besonders bevorzugt verwendet man Polyphenyl-polymethylen-polyisocyanate (Polymer-MDI, PMDI) mit zwei oder mehr aromatischen Systemen, Mischungen aus 2,4'-, 2,2'-, und/oder 4,4'-Diphenylmethan-diisocyanaten und Polyphenyl-polymethylen-polyisocyanaten (Roh-MDI), Mischungen aus Roh-MDI und Toluylen-Diisocyanaten und Polyphenyl-Polyisocyanate.

Häufig werden auch sogenannte modifizierte mehrwertige Isocyanate, d.h. Produkte, die durch partielle chemische Umsetzung organischer Di- und/oder Polyisocyanate erhalten werden, verwendet. Beispielhaft genannt seien Ester-, Harnstoff-, Biuret-, Allophanat-, Carbodiimid-, Isocyanurat- und/oder Urethangruppen enthaltende Di- und/oder Polyisocyanate. Im einzelnen kommen beispielsweise in Betracht: Urethangruppen enthaltende organische, vorzugsweise aromatische Polyisocyanate mit NCO-Gehalten von 33,6 bis 15 Gew.-%, vorzugsweise von 31 bis 21 Gew.-%, bezogen auf das Gesamtgewicht, beispielsweise mit niedermolekularen Diolen, Triolen, Dialkylenglykolen, Trialkylenglykolen oder Polyoxyalkylenglykolen mit Molekulargewichten bis 1500 modifiziertes 4,4'-Diphenylmethan-diisocyanat oder 2,4- bzw. 2,6-Toluylen-diisocyanat, wobei als Di- bzw. Polyoxyalkylenglykole, die einzeln oder als Gemische eingesetzt werden können; beispielsweise genannt seien: Diethylen-, Dipropylen-glykol, Polyoxyethylen-, Polyoxypropylen- und Polyoxypropylen-polyoxyethylen-glykole oder -triole. Geeignet sind auch NCO-Gruppen enthaltende Prepolymere mit NCO-Gehalten von 25 bis 9 Gew.-%, vorzugsweise von 21 bis 14 Gew.-%, bezogen auf das Gesamtgewicht, hergestellt aus den nachfolgend beschriebenen Polyester-und/oder vorzugsweise Polyether-polyolen und 4,4'-Diphenylmethan-diisocyanat, Mischungen aus 2,4'- und 4,4'-Diphenyl-methan-diisocyanat, 2,4- und/oder 2,6-Toluylen-diisocyanaten oder Roh-MDI. Bewährt haben sich ferner flüssige, Carbodiimidgruppen und/oder Isocyanuratringe enthaltende Polyisocyanate mit NCO-Gehalten von 33,6 bis 15, vorzugsweise 31 bis 21 Gew.-%, bezogen auf das Gesamtgewicht, z.B. auf Basis von 4,4'-, 2,4'- und/oder 2,2'-Diphenylmethan-diiso-cyanat und/oder 2,4- und/oder 2,6-Toluylen-diisocyanat.

Die modifizierten Polyisocyanate können miteinander oder mit unmodifizierten organischen Polyisocyanaten wie z.B. 2,4'-, 4,4'-Diphenylmethan-diisocyanat, Roh-MDI, 2,4- und/oder 2,6-Toluylen-diiso-cyanat gegebenenfalls gemischt werden.

Besonders bewährt haben sich als organische Polyisocyanate und kommen daher vorzugsweise zur Anwendung zur Herstellung der PU-Hartschaumstoffe: Mischungen aus Toluylen-diisocyanaten und Roh-MDI oder Mischungen aus modifizierten Urethangruppen enthaltenden organischen Polyisocyanaten mit einem NCO-Gehalt von 33,6 bis 15 Gew.-%, insbesondere solchen auf Basis von Toluylen-diisocyanaten, 4,4'-Diphenyl-methan-diisocyanat, Diphenylmethan-diisocyanat-Isomerengemischen oder Roh-MDI und insbesondere Roh-MDI mit einem Diphenylmethan-diisocyanat-Isomerengehalt von 30 bis 80 Gew.-%, vorzugsweise von 30 bis 55 Gew.-%.

Die (a) Polyisocyanate kann man unter anderem einsetzen zur allgemein bekannten Herstellung von Polyisocyanat-Polyadditionsprodukten wie z.B. Polyurethanen oder/und Polyisocyanuraten. Dafür werden die Polyisocyanate oder Polyisocyanatkomponenten mit (b) gegenüber Isocyanaten reaktiven Substanzen sowie gegebenenfalls (c) Kettenverlängerungs- und/oder Vernetzungsmitteln, (d) Treibmitteln, (e) Katalysatoren und (f) Hilfs- und Zusatzstoffen in Gegenwart der erfindungsgemäßen Zinnverbindungen umgesetzt. Zu den Komponenten (b) bis (f) wird im einzelnen folgendes ausgeführt:
b) Als Verbindungen mit mindestens zwei reaktiven Wasserstoffatomen (b) kommen vorzugsweise Polyhydroxylverbindungen mit einer Funktionalität von 2 bis 8, vorzugsweise von 3 bis 8 und einer Hydroxylzahl von 150 bis 850, vorzugsweise von 350 bis 800 in Betracht.
   Beispielhaft genannt seien Polythioether-polyole, Polyesteramide, hydroxylgruppenhaltige Polyacetale, hydroxylgruppenhaltige aliphatische Polycarbonate und vorzugsweise Polyester-polyole und Polyether-polyole. Anwendung finden auch Mischungen aus mindestens zwei der genannten Polyhydroxylverbindungen, sofern diese eine durchschnittliche Hydroxylzahl im vorgenannten Bereich aufweisen.
   Geeignete Polyester-polyole, können beispielsweise aus organischen Dicarbonsäuren mit 2 bis 12 Kohlenstoffatomen, vorzugsweise aliphatischen Dicarbonsäuren mit 4 bis 6 Kohlenstoffatomen und mehrwertigen Alkoholen, vorzugsweise Diolen, mit 2 bis 12 Kohlenstoffatomen, vorzugsweise 2 bis 6 Kohlenstoffatomen hergestellt werden. Als Dicarbonsäuren kommen beispielsweise in Betracht: Bernsteinsäure, Glutarsäure, Adipinsäure, Korksäure, Azelainsäure, Sebacinsäure, Decandicarbon-Säure, Maleinsäure, Fumarsäure, Phthalsäure, Isophthalsäure und Terephthalsäure. Die Dicarbonsäuren können dabei sowohl einzeln als auch im Gemisch untereinander verwendet werden. Anstelle der freien Dicarbonsäuren können auch die entsprechenden Dicarbonsäurederivate, wie z.B. Dicarbonsäuremono- oder -diester von Alkoholen mit 1 bis 4 Kohlenstoffatomen oder Dicarbonsäureanhydride eingesetzt werden. Vorzugsweise verwendet werden Dicarbonsäuregemische aus Bernstein-, Glutar- und Adipinsäure in Mengenverhältnissen von beispielsweise 20 bis 35 : 35 bis 50 : 20 bis 32 Gew.-Teilen, und insbesondere Adipinsäure. Beispiele für zwei- und mehrwertige Alkohole, insbesondere Diole sind: Ethandiol, Diethylenglykol, 1,2- bzw. 1,3-Propandiol, Di-propylenglykol, 1,4-Butandiol, 1,5-Pentandiol, 1,6-Hexandiol, 1,10-Decandiol, Glycerin und Trimethylolpropan. Vorzugsweise verwendet werden Ethandiol, Diethylenglykol, 1,4-Butandiol, 1,5-Pentandiol, 1,6-Hexandiol oder Mischungen aus mindestens zwei der genannten Diole, insbesondere Mischungen aus 1,4-Butandiol, 1,5-Pentandiol und 1,6-Hexandiol. Eingesetzt werden können ferner Polyester-polyole aus Lactonen, z.B ε-Caprolacton oder Hydroxycarbonsäuren, z.B. ω-Hydroxycapronsäure.
   Zur Herstellung der Polyester-polyole können die organischen, z.B. aromatischen und vorzugsweise aliphatischen Polycarbonsäuren und/oder -derivate und mehrwertiger Alkohole katalysatorfrei oder vorzugsweise in Gegenwart von Veresterungskatalysatoren, zweckmäßigerweise in einer Atmosphäre aus Inertgasen, wie z.B. Stickstoff, Kohlendioxid, Helium, Argon u.a. in der Schmelze bei Temperaturen von 150 bis 250°C, vorzugsweise 180 bis 220°c gegebenenfalls unter vermindertem Druck bis zu der gewünschten Säurezahl, die vorteilhafterweise kleiner als 10, vorzugsweise kleiner als 2 ist, polykondensiert werden. Nach einer bevorzugten Ausführungsform wird das Veresterungsgemisch bei den obengenannten Temperaturen bis zu einer Säurezahl von 80 bis 30, vorzugsweise 40 bis 30, unter Normaldruck und anschließend unter einem Druck von kleiner als 500 mbar, vorzugsweise 50 bis 150 mbar, polykondensiert. Als Veresterungskatalysatoren kommen beispielsweise Eisen-, Cadmium-, Kobalt-, Blei-, Zink-, Antimon-, Magnesium-, Titan- und Zinnkatalysatoren, in Form von Metallen, Metalloxiden oder Metallsalzen in Betracht. Die Polykondensation kann jedoch auch in flüssiger Phase in Gegenwart von Verdünnungs und/oder Schleppmitteln, wie z.B. Benzol, Toluol, Xylol oder Chlorbenzol, zur azeotropen Abdestillation des Kondensationswassers durchgeführt werden.
   Zur Herstellung der Polyester-polyole werden die organischen Polycarbonsäuren und/oder -derivate und mehrwertigen Alkohole üblicherweise im Molverhältnis von 1:1 bis 1,8, vorzugsweise 1:1,05 bis 1,2 polykondensiert.
   Die erhaltenen Polyester-polyole besitzen vorzugsweise eine Funktionalität von 2 bis 3 und eine Hydroxylzahl von 150 bis 400 und insbesondere von 200 bis 300.
   Insbesondere als Polyhydroxylverbindungen verwendet werden jedoch Polyether-polyole, die nach bekannten Verfahren, beispielsweise durch anionische Polymerisation mit Alkalihydroxiden, wie Natrium- oder Kaliumhydroxid oder Alkalialkoholaten, wie Natriummethylat, Natrium- oder Kaliumethylat oder Kaliumisopropylat als Katalysatoren und mindestens einem Startermolekül, das 2 bis 8, vorzugsweise 3 bis 8 reaktive Wasserstoffatome gebunden enthält, oder durch kationische Polymerisation mit Lewis-Säuren, wie Antimonpentachlorid, Borfluorid-Etherat u.a. oder Bleicherde als Katalysatoren aus einem oder mehreren Alkylenoxiden mit 2 bis 4 Kohlenstoffatomen im Alkylenrest hergestellt werden.
   Geeignete Alkylenoxide sind beispielsweise Tetrahydrofuran, 1,3-Propylenoxid, 1,2- bzw. 2,3-Butylenoxid, Styroloxid und vorzugsweise Ethylenoxid und 1,2-Propylenoxid. Die Alkylenoxide können einzeln, alternierend nacheinander oder als Mischungen verwendet werden. Als Startermoleküle kommen beispielsweise in Betracht: Wasser, organische Dicarbonsäuren, wie Bernsteinsäure, Adipinsäure, Phthalsäure und Terephthalsäure, aliphatische und aromatische, gegebenenfalls N-mono-, N,N- und N,N'-dialkylsubstituierte Diamine mit 1 bis 4 Kohlenstoffatomen im Alkylrest, wie gegebenenfalls mono- und dialkylsubstituiertes Ethylendiamin, Diethylentriamin, Triethylentetramin, 1,3-Propylen-diamin, 1,3- bzw. 1,4-Butylendiamin, 1,2-, 1,3-, 1,4-, 1,5- und 1,6-Hexamethylendiamin, Phenylendiamine, 2,3-, 2,4- und 2,6-Toluylen-diamin und 4,4'-, 2,4'- und 2,2'-Diamino-diphenylmethan.
   Als Startermoleküle kommen ferner in Betracht: Alkanolamine, wie Ethanolamin, Diethanolamin, N-Methyl- und N-Ethyl-ethanolamin, N-Methyl- und N-Ethyl-diethanolamin und Triethanolamin und Ammoniak.
   Vorzugsweise verwendet werden mehrwertige, insbesondere drei- und/oder höherwertige Alkohole, wie Ethandiol, Propandiol-1,2 und -1,3, Diethylenglykol, Dipropylenglykol, Butandiol-1,4, Hexandiol-1,6, Glycerin, Trimethylol-propan, Pentaerythrit, Sorbit und Sucrose.
   Die Polyether-polyole besitzen eine Funktionalität von vorzugsweise 3 bis 8 und insbesondere 3 und 6 und Hydroxylzahlen von vorzugsweise 300 bis 850 und insbesondere von 350 bis 800.
   Als Polyether-polyole eignen sich ferner Melamin-Polyetherpolyole-Dispersionen gemäß EP-A-23 987 (US-A-4 293 657), Polymer-Polyether-Polyol-Dispersionen, hergestellt aus Polyepoxiden und Epoxidharzhärtern in Gegenwart von Polyetherpolyolen gemäß DE 29 43 689 (US 43 05 861), Dispersionen von aromatischen Polyestern in Polyhydroxylverbindungen gemäß EP-A-62 204 (US-A-44 35 537) oder DE-A 33 00 474, Dispersionen von organischen und/oder anorganischen Füllstoffen in Polyhydroxylverbindungen gemäß EP-A-11 751 (US 42 43 755), Polyharnstoff-Polyether-polyol-Dispersionen gemäß DE-A-31 25 402, Tris-(hydroxyalkyl)isocyanurat-Polyether-polyol-Dispersionen gemäß EP-A-136 571 (US 4 514 526) und Kristallitsuspensionen gemäß DE-A-33 42 176 und DE-A-33 42 177 (US 45 60 708), wobei die Ausführungen in den genannten Patentveröffentlichungen als Bestandteil der Patentbeschreibung zu betrachten sind.
   Die Polyether-polyole können ebenso wie die Polyester-polyole einzeln oder in Form von Mischungen verwendet werden. Ferner können sie mit den obengenannten Dispersionen, Suspensionen oder Polyester-polyolen sowie den hydroxylgruppenhaltigen Polyesteramiden, Polyacetalen und/oder Polycarbonaten gemischt werden.
   Als hydroxylgruppenhaltige Polyacetale kommen z.B. die aus Glykolen, wie Diethylenglykol, Triethylenglykol, 4,4-Dihydroxyethoxydiphenyl-dimethylmethan, Hexandiol und Formaldehyd herstellbaren Verbindungen in Frage. Auch durch Polymerisation cyclischer Acetale lassen sich geeignete Polyacetale herstellen.
   Als Hydroxylgruppen aufweisende Polycarbonate kommen solche der an sich bekannten Art in Betracht, die beispielsweise durch Umsetzung von Diolen, wie Propandiol-(1,3), Butandiol-(1,4) und/oder Hexandiol-(1,6), Diethylenglykol, Triethylenglykol oder Tetraethylenglykol mit Diarylcarbonaten, z.B. Diphenylcarbonat, oder Phosgen hergestellt werden können.
   Zu den Polyesteramiden zählen z.B. die aus mehrwertigen gesättigten und/oder ungesättigten Carbonsäuren bzw. deren Anhydriden und Aminoalkoholen oder Mischungen aus mehrwertigen Alkoholen und Aminoalkoholen und/oder Polyaminen gewonnenen, vorwiegend linearen Kondensate.
   Als Polyhydroxylverbindungen besonders bewährt haben sich und daher vorzugsweise verwendet werden Mischungen, die bezogen auf 100 Gew.-Teile, zweckmäßigerweise enthalten:
   - bi): 0 bis 95 Gew.-Teile, vorzugsweise 20 bis 80 Gew.-Teile eines mit Sucrose gestarteten Polyether-polyols mit einer Hydroxylzahl von 300 bis 500, vorzugsweise 350 bis 450 auf der Grundlage von 1,2-Propylenoxid oder 1,2-Propylenoxid und Ethylenoxid,
   - bii): 0 bis 80 Gew.-Teile, vorzugsweise 5 bis 25 Gew.-Teile eines mit Sorbit gestarteten Polyether-polyols mit einer Hydroxylzahl von 400 bis 600, vorzugsweise von 450 bis 550 auf der Grundlage von 1,2-Propylenoxid oder 1,2-Propylenoxid und Ethylenoxid,
   - biii): 0 bis 20 Gew.-Teile, vorzugsweise 5 bis 15 Gew.-Teile eines mit Ethylendiamin gestarteten Polyether-polyols mit einer Hydroxylzahl von 700 bis 850, vorzugsweise von 750 bis 800 auf der Grundlage von 1,2-Propylenoxid
   und
   - biiii): 0 bis 60 Gew.-Teile, vorzugsweise 5 bis 40 Gew.-Teile eines Polyether-polyols mit einer Hydroxylzahl von 400 bis 600, vorzugsweise von 450 bis 550 auf Basis von 1,2-Propylenoxid oder 1,2-Propylenoxid und Ethylenoxid, hergestellt unter Verwendung einer Mischung aus Sucrose und Triethanolamin im Gewichtsverhältnis von 1:2 bis 2:1 als Startermoleküle.
c) Die PU-Hartschaumstoffe können ohne oder unter Mitverwendung von Kettenverlängerungs- und/oder Vernetzungsmitteln hergestellt werden. Zur Modifizierung der mechanischen Eigenschaften kann sich jedoch der Zusatz von Kettenverlängerungsmitteln, Vernetzungsmitteln oder gegebenenfalls auch Gemischen davon als vorteilhaft erweisen. Als Kettenverlängerungs- und/oder Vernetzungsmittel kann man vorzugsweise Alkanolamine und insbesondere Wasser, Diole und/oder Triole mit Molekulargewichten kleiner als 400, vorzugsweise von 60 bis 300 verwenden. In Betracht kommen beispielsweise Alkanolamine, wie z.B. Ethanolamin und/oder Isopropanolamin, Dialkanolamine wie z.B. Diethanolamin, N-Methyl-, N-Ethyldiethanolamin, Diisopropanolamin, Trialkanolamine wie z.B Triethanolamin, Triisopropanolamin und die Additionsprodukte aus Ethylenoxid oder 1,2-Propylenoxid und Alkylendiaminen mit 2 bis 6 C-Atomen im Alkylenrest wie z.B N,N'-Tetra(2-hydroxyethyl)ethylendiamin und N,N'-Tetra(2-hydroxypropyl)ethylendiamin, aliphatische, cycloaliphatische und/oder araliphatische Diole mit 2 bis 14, vorzugsweise 4 bis 10 Kohlenstoffatomen, wie z.B Ethylenglykol, Propan-diol-1,3, Decandiol-1,10, o-, m-, p-Dihydroxycyclohexan, Diethylenglykol, Dipropylenglykol und vorzugsweise Butandiol-1,4, Hexandiol-1,6 und Bis-(2-hydroxyethyl)-hydrochinon, Triole, wie 1,2,4-, 1,3,5-Tri- hydroxy-cyclohexan, Glycerin und Trimethylolpropan und niedermolekulare hydroxylgruppenhaltige Polyalkylenoxide auf Basis Ethylen- und/oder 1,2-Propylenoxid und aromatischen Diaminen, wie z.B Toluylen-diaminen und/oder Diamino-diphenylmethanen sowie den vorgenannten Alkanolaminen, Diolen und/oder Triolen als Startermoleküle.
   Sofern zur Herstellung der PU-Hartschaumstoffe Kettenverlängerungsmittel, Vernetzungsmittel oder Mischungen davon Anwendung finden, kommen diese zweckmäßigerweise in einer Menge von 0 bis 20 Gew.-%, vorzugsweise von 2 bis 5 Gew.-%, bezogen auf das Gewicht der Polyhydroxylverbindung zum Einsatz.
d) Als Treibmittel können allgemein bekannte Treibmittel, wie z.B. Stoffe, die einen Siedepunkt unter Normaldruck im Bereich von -40°C bis 120°C besitzen, Gase und/oder feste Treibmittel und/oder Wasser eingesetzt werden, beispielsweise Kohlendioxid, Alkane und oder Cycloalkane wie beispielsweise Isobutan, Propan, n- oder iso-Butan, n-Pentan und Cyclopentan, Ether wie beispielsweise Diethylether, Methylisobutylether und Dimethylether, Stickstoff, Sauerstoff, Helium, Argon, Lachgas, halogenierte Kohlenwasserstoffe, beispielsweise Dichlorfluormethan, Monofluortrichlor-methan, Trifluortrichlorethan und/oder teilhalogenierte Kohlenwasserstoffe wie beispielsweise Trifluormethan, 1,1-Dichlor-1-fluorethan, Monochlortetrafluorethan, Monochlortrifluorethan, Monochlordifluorethan, Difluorethan, Dichlordifluorethan, Pentafluorethan, Tetrafluorethan, Dichlormonofluorethan oder Mischungen, die mindestens zwei der beispielhaft genannten Treibmittel enthalten.
   Als Treibmittel zur Herstellung der PU-Hartschaumstoffe findet erfindungsgemäß vorzugsweise Cyclopentan Verwendung.
e) Als übliche Katalysatoren (e) können zusätzlich zu den erfindungsgemäßen Zinnverbindungen allgemein bekannte stark basische Stickstoff-haltige Verbindungen eingesetzt werden. Genannt seien beispielsweise Amidine, wie 2,3-Dimethyl-3,4,5,6-tetrahydropyrimidin, tertiäre Amine, wie Triethylamin, Tributylamin, Dimethylbenzylamin, N-Methyl-, N-Ethyl-, N-Cyclohexylmorpholin, N,N,N',N'-Tetramethyl-ethylendiamin, N,N,N',N'-Tetramethyl-butandiamin oder -hexandiamin, Pentamethyl-diethylentriamin, Tetramethyl-diaminoethylether, Bis-(di-methylaminopropyl)-harnstoff, Dimethylpiperazin, 1,2-Dimethylimidazol, 1-Azabicyclo-(3,3,0)-octan und vorzugsweise 1,4-Diaza-bicyclo-(2,2,2)-octan und Alkanolaminverbindungen, wie Triethanolamin, Tri-isopropanolamin, N-Methyl-und N-Ethyl-diethanolamin und Dimethylethanolamin.
   Als Katalysatoren kommen ferner in Betracht: Tris-(dialkylaminoalkyl)-s-hexahydrotriazine, insbesondere Tris-(N,N-dimethylaminopropyl)-s-hexahydrotriazin, Tetraalkylammoniumhydroxide, wie Tetramethylammoniumhydroxid, Alkalihydroxide, wie Natriumhydroxid und Alkalialkoholate, wie Natriummethylat und Kaliumisopropylat, Alkalisalze von langkettigen Fettsäuren mit 10 bis 20 C-Atomen und gegebenenfalls seitenständigen OH-Gruppen sowie übliche Katalysatoren, die die Bildung von PIR beschleunigen, wie z.B. Kaliumacetat.
   Vorzugsweise verwendet werden 0,001 bis 5 Gew.-%, insbesondere 0,05 bis 2 Gew.-%, bezogen auf das Gewicht der Komponente (b), dieser zusätzlich zu den erfindungsgemäßen Zinn-Katalysatoren einzusetzenden Katalysatoren.
f) Der Reaktionsmischung zur Herstellung der PU-Hartschaumstoffe können gegebenenfalls auch noch Hilfsmittel und/oder Zusatzstoffe (f) einverleibt werden. Genannt seien beispielsweise oberflächenaktive Substanzen, Schaumstabilisatoren, Zellregler, Füllstoffe, Farbstoffe, Pigmente, Flammschutzmittel, Hydrolyseschutzmittel, fungistatische und bakteriostatisch wirkende Substanzen.
   Als oberflächenaktive Substanzen kommen z.B. Verbindungen in Betracht, welche zur Unterstützung der Homogenisierung der Ausgangsstoffe dienen und gegebenenfalls auch geeignet sind, die Zellstruktur zu regulieren. Genannt seien beispielsweise Emulgatoren, wie die Natriumsalze von Ricinusölsulfaten, oder von Fettsäuren sowie Salze von Fettsäuren mit Aminen, z.B. ölsaures Diethylamin, stearinsaures Diethanolamin, ricinolsaures Diethanolamin, Salze von Sulfonsäuren, z.B. Alkali- oder Ammoniumsalze von Dodecylbenzol- oder Dinaphthylmethandisulfonsäure und Ricinolsäure; Schaumstabilisatoren, wie Siloxan-Oxalkylen-Mischpolymerisate und andere Organopolysiloxane, oxethylierte Alkylphenole, oxethylierte Fettalkohole, Paraffinöle, Ricinusöl- bzw. Ricinolsäureester, Türkischrotöl und Erdnußöl und Zellregler, wie Paraffine, Fettalkohole und Dimethylpolysiloxane. Zur Verbesserung der Emulgierwirkung, der Zellstruktur und/oder Stabilisierung des Schaumes eignen sich ferner oligomere Polyacrylate mit Polyoxyalkylen- und Fluoralkanresten als Seitengruppen. Die oberflächenaktiven Substanzen werden üblicherweise in Mengen von 0,01 bis 5 Gew.-Teilen, bezogen auf 100 Gew.-Teile der Komponente (b), angewandt.
   Als Füllstoffe, insbesondere verstärkend wirkende Füllstoffe, sind die an sich bekannten, üblichen organischen und anorganischen Füllstoffe, Verstärkungsmittel, Beschwerungsmittel, Mittel zur Verbesserung des Abriebverhaltens in Anstrichfarben, Beschichtungsmittel usw. zu verstehen. Im einzelnen seien beispielhaft genannt: anorganische Füllstoffe wie silikatische Mineralien, beispielsweise Schichtsilikate wie Antigorit, Serpentin, Hornblenden, Amphibole, Chrisotil, Talkum; Metalloxide, wie Kaolin, Aluminiumoxide, Aluminiumsilikat, Titanoxide und Eisenoxide, Metallsalze wie Kreide, Schwerspat und anorganische Pigmente, wie Cadmiumsulfid, Zinksulfid sowie Glaspartikel. Als organische Füllstoffe kommen beispielsweise in Betracht: Ruß, Melamin, Kollophonium, Cyclopentadienylharze und Pfropfpolymerisate.
   Die anorganischen und organischen Füllstoffe können einzeln oder als Gemische verwendet werden und werden der Reaktionsmischung vorteilhafterweise in Mengen von 0,5 bis 50 Gew.-%, vorzugsweise 1 bis 40 Gew.-%, bezogen auf das Gewicht der Komponenten (a) bis (c), einverleibt.
   Geeignete Flammschutzmittel sind beispielsweise Trikresylphosphat, Tris-(2-chlorethyl)phosphat, Tris-(2-chlorpropyl)phosphat, Tris(1,3-dichlorpropyl)phosphat, Tris-(2,3-dibrompropyl)phosphat und Tetrakis-(2-chlorethyl)-ethylendiphosphat.
   Außer den bereits genannten halogensubstituierten Phosphaten können auch anorganische Flammschutzmittel, wie roter Phosphor, Aluminiumoxidhydrat, Antimontrioxid, Arsenoxid, Ammoniumpolyphosphat und Calciumsulfat oder Cyanursäurederivate, wie z.B. Melamin oder Mischungen aus mindestens zwei Flammschutzmitteln, wie z.B. Ammoniumpolyphosphaten und Melamin sowie gegebenenfalls Stärke zum Flammfestmachen der erfindungsgemäß hergestellten PU-Hartschaumstoffe verwendet werden. Im allgemeinen hat es sich als zweckmäßig erwiesen, 5 bis 50 Gew.-Teile, vorzugsweise 5 bis 25 Gew.-Teile der genannten Flammschutzmittel oder -mischungen für jeweils 100 Gew.-Teile der Komponenten (a) bis (c) zu verwenden.
   Nähere Angaben über die oben genannten anderen üblichen Hilfs- und Zusatzstoffe sind der Fachliteratur, beispielsweise der Monographie von J.H. Saunders und K.C. Frisch "High Polymers" Band XVI, Polyurethanes, Teil 1 und 2, Verlag Interscience Publishers 1962 bzw. 1964, oder dem Kunststoff-Handbuch, Polyurethane, Band VI I, Carl-Hanser-Verlag, München, Wien, 1. und 2. Auflage, 1966 und 1983 zu entnehmen.

Zur Herstellung der PU-Hartschaumstoffe werden die organischen, gegebenenfalls modifizierten Polyisocyanate (a), die höhermolekularen Verbindungen mit mindestens zwei reaktiven Wasserstoffatomen (b) und gegebenenfalls Kettenverlängerungsmittel und/ oder Vernetzungsmittel (c) in solchen Mengen zur Umsetzung gebracht, daß das Äquivalenz-Verhältnis von NCO-Gruppen der Polyisocyanate (a) zur Summe der reaktiven Wasserstoffatome der Komponenten (b) und gegebenenfalls (c) 0,85 bis 1,80:1, vorzugsweise 1,1 bis 1,4:1 und insbesondere ungefähr 1,15 bis 1,3:1 beträgt. Sofern die Urethangruppen enthaltende Schaumstoffe durch die Bildung von Isocyanuratgruppen modifiziert werden, beispielsweise zur Erhöhung der Flammwidrigkeit, wird üblicherweise ein Verhältnis von NCO-Gruppen der Polyisocyanate (a) zur Summe der reaktiven Wasserstoffatome der Komponente (b) und gegebenenfalls (c) von 1,5 bis 10:1, vorzugsweise 1,5 bis 6:1 angewandt.

Die PU-Hartschaumstoffe können diskontinuierlich oder kontinuierlich nach dem Prepolymer- oder vorzugsweise nach dem one shot-Verfahren mit Hilfe bekannter Mischvorrichtungen z.B. in geschlossenen Formwerkzeugen oder beispielsweise mittels eines Doppeltransportbandes hergestellt werden.

Als besonders vorteilhaft hat es sich erwiesen, nach dem Zweikomponenten-Verfahren zu arbeiten und die Aufbaukomponente (b), (d), (e) und gegebenenfalls (c) und (f) in der Komponente (A) zu vereinigen und als Komponente (B) die organischen Polyisocyanate, modifizierten Polyisocyanate (a) oder Mischungen aus den genannten Polyisocyanaten, die erfindungsgemäßen Zinnverbindungen und gegebenenfalls Treibmittel (d) zu verwenden.

Die Ausgangskomponenten werden bei einer Temperatur von 15 bis 90°C, vorzugsweise von 20 bis 35°C gemischt und in ein offenes, gegebenenfalls temperiertes Formwerkzeug eingebracht, in der man die Reaktionsmischung zur Vermeidung einer verdichteten Randzone im wesentlichen druckfrei aufschäumen läßt. Zur Bildung von Verbundelementen beschichtet man zweckmäßigerweise die Rückseite einer Deckschicht, z.B. durch Begießen oder Besprühen, mit der schaumfähigen Reaktionsmischung und lädt diese aufschäumen und zum PU-Hartschaumstoff aushärten.

Bevorzugte Verwendung finden die Polyisocyanate in der Herstellung von PUR oder PUR/PIR-Hartschaumstoffen mit einer bevorzugten Dichte von 10 bis 200 kg/m³.

Besonders vorteilhaft ist der Einsatz der Polyisocyanate zur Herstellung von PUR- oder PUR/PIR-Hartschaumstoffen mit einem Mindestdurchmesser von 40 bis 2000 mm, insbesondere von 250 bis 1500 mm, wobei sich der Mindestdurchmesser durch den Durchmesser der größten in die Form des Hartschaumstoffes einschreibbaren Kugel ergibt, d.h., durch den Durchmesser der größten Kugel, die in die Formen der Polyurethane und/oder Polyisocyanurate paßt.

Die PU-Hartschaumstoffe finden vorzugsweise Verwendung als wärmedämmende Zwischenschicht in Verbundelementen und zum Ausschäumen von Hohlräumen in Kühlmöbelgehäusen, insbesondere für Kühlschränke und Gefriertruhen, und als Außenmantel von Heißwasserspeichern. Die Produkte eignen sich ferner zur Isolierung von erwärmten Materialien, als Motorabdeckung und als Rohrschalen oder auch als konstruktiv einsetzbare Sandwichelemente.

Die Vorteile der erfindungsgemäßen Verwendung der Polyisocyanate bestehen unter anderem in ihrer sehr guten Lagerfähigkeit und bei der Herstellung von PUR- oder PUR/PIR-Hartschaumstoffen in der verminderten Rißbildung der Schaumstoffe, besonders bei Schäumen mit großer Dicke. Diese Vorteile sollen anhand der folgenden Beispiele dargestellt werden.

Die Beispiele basieren auf einem Gemisch von Diphenylmethandiisocyanat und Polyphenyl-polymethylen-polyisocyanaten (Roh-MDI) mit den folgenden Kennwerten:

**Tabelle 1:**

| Isocyanat: | (a) | (b) | (c) |
|---|---|---|---|
| Isocyanantgruppen [%]: | 31,7 | 31,7 | 30,7 |
| Isocyanate mit zwei Aromaten im Molekül [%]: | 45,7 | 43,8 | 30,4 |
| Isocyanate mit drei Aromaten im Molekül [%] : | 19.9 | 20,3 | 27,2 |
| Isocyanate mit vier Aromaten im Molekül [%]: | 10,3 | 10,6 | 12,0 |
| Isocyanate mit mehr als vier Aromaten im Molekül [%]: | 24,1 | 25,3 | 30,4 |
| Summe leicht- und schwer hydrolysierbares Chlor [ppm] : DHC | 1176 | 1018 | 1352 |
| Gehalt an Eisen [ppm]: | 1,1 | 7,0 | 4,5 |
| Viskosität bei 25°C [mPas] | 209 | 170 | 625 |

Das Diphenylmethandiisocyanat setzte sich für das Isocyanat (a) anteilig aus den folgenden Isomeren zusammen:
2,2'-Diphenylmethandiisocyanat: 0,58 %,
2,4'-Diphenylmethandiisocyanat: 10,87 %,
4,4'-Diphenylmethandiisocyanat: 88,55 %.

Für die erfindungsgemäßen (gekennzeichnet mit e) bzw. Vergleichsbeispiele (v) erfolgte eine Zugabe der folgenden Katalysatoren in den angegebenen Mengen an Eisen oder Zinn zu den Isocyanaten (a) oder (b):

**Tabelle 2:**

| Beispiel | Katalysator | Katalysatorzugabe als Fe bzw. Sn [ppm] | Isocyanat |
|---|---|---|---|
| 1e | Dibutylzinndilaurat | 6,56 | (a) |
| 2e | Dibutylzinndilaurat | 7,49 | (a) |
| 3e | Dibutylzinndilaurat | 8,43 | (a) |
| 4e | Dibutylzinndilaurat | 9,74 | (a) |
| 5e | Dibutylzinndilaurat | 12 | (a) |
| 6e | Dibutylzinndilaurat | 8,43 | (b) |
| 7e | Dibutylzinndilaurat | 9,74 | (b) |
| 8e | Zinn-II-octoat | 4,4 | (a) |
| 9e | Zinn-II-octoat | 10,3 | (a) |
| 10e | Zinn-II-octoat | 17,6 | (a) |
| 11v | Eisen-III-chlorid | 5 | (a) |
| 12v | Eisen-III-chlorid | 7 | (a) |
| 13v | Eisen-III-chlorid | 10 | (a) |
| 14v | Eisen-III-chlorid | 20 | (a) |
| 15v | Eisen-III-chlorid | 40 | (a) |
| 16v | Eisen-III-chlorid | 21 | (b) |
| 17v | Zinn-II-octoat | 30 | (a) |
| 18v | Zinn-II-octoat | 50 | (a) |
| 19v | Zinn-II-octoat | 100 | (a) |
| 20e | Di-n-octylzinnmercaptid | 4 | (c) |

Diese Polyisocyanate wurden in den Beispielen hinsichtlich der folgenden Eigenschaften untersucht:
i) Reaktivität gegenüber Polyhydroxylverbindungen
ii) Charakterisierung der Rißbildung bei der Herstellung von Formkörpern aus PUR-Hartschaumstoffen

### i) Reaktivität gegenüber Polyhydroxylverbindungen:

Die Charakterisierungen der Reaktivität der Polyisocyanate (B-Komponente) wurden mit einer Polyhydroxylkomponente (A-Komponente), die aus:

| | |
|---|---|
| 92 Gew.-% | einer Polyhydroxylverbindung, hergestellt aus 1,1,1-Trimethylolpropan und Propylenoxid, mit einer Hydroxylzahl von 555 mg KOH/g, |
| | |
| 3 Gew.-% | einer Polyhydroxylverbindung, hergestellt aus Ethylendiamin und Propylenoxid und |
| | |
| 5 Gew.-% | Zeolith, 40 nm Porengröße, 50 %-ige Lösung in Ricinusöl |

bestand, durchgeführt.

100 g der der (A)-Komponente, temperiert auf 25 ± 0,5°C, wurden mit 140 g der (B)-Komponente (25 ± 0,5°C) in einem Becher aus Pappe mit 660 ml Rauminhalt 15 s mit einem Laborrührwerk, das mit einem Rührorgan der Fa. Vollrath ausgestattet war, mit 1750 U/min verrührt. Mit einem Thermoelement, das 1 cm über dem Becherboden angebracht war, wurde der Temperaturverlauf der Reaktionsmischung von Beginn der Vermischung an gemessen. Die Zeit, die das Reaktionsgemisch für den Temperaturanstieg auf 90°C benötigte, wird im Folgenden als Reaktionszeit bezeichnet und kann als Maß für die Reaktivität der Polyisocyanate gewertet werden. Liegt dieser Wert zwischen 300 und 500 s, bevorzugt zwischen 400 und 500 s, so ist Polyisocyanat zur Herstellung von Hartschaum-Blockware einsetzbar. Die angegebenen Reaktionszeiten stellt den Mittelwert von drei Messungen dar. Des weiteren ist die Standardabweichung angegeben. Zur Bestimmung der Lagerstabilität wurden die Isocyanatgehalte sowie entsprechend der obigen Ausführungen die Reaktionszeiten der Polyisocyanate nach Lagerung bei Raumtemperatur in 2,5 l Aluminium-Gebinden unter Luftabschluß bestimmt. Die Messungen, die ohne Lagerung der Polyisocyanate durchgeführt wurden, sind mit einer Lagerzeit von '0' gekennzeichnet. Die Ergebnisse sind in Tabelle 3 dargestellt.

**Tabelle 3:**

| Polyisocyanat | Lagerzeit | Reaktionszeit [s] | Isocyanatgehalt [%] |
|---|---|---|---|
| 6e | 1 Monat | 480 ± 23 | 31,7 |
| 6e | 2 Monate | 488 ± 18 | 31,8 |
| 6e | 3 Monate | 499 ± 22 | 31,7 |
| 6e | 5 Monate | 423 ± 18 | 31,8 |
| 6e | 8 Monate | 481 ± 2 | 31,7 |
| 7e | 0 | 422 ± 11 | 31,7 |
| 7e | 1 Monat | 430 ± 9 | 31,7 |
| 7e | 3 Monate | 460 ± 16 | 31,7 |
| 7e | 6 Monate | 418 ± 3 | 31,4 |
| 16v | 0 | 467 ± 20 | 31,3 |
| 16v | 1 Monat | 493 ± 1 | 31,5 |
| 16v | 2 Monate | 499 ± 22 | 31,3 |
| 16v | 3 Monate | 508 ± 18 | 31,8 |
| 16v | 4 Monate | 448 ± 3 | 31,5 |
| 16v | 5 Monate | 398 ± 5 | 31,7 |
| 16v | 8 Monate | 332 ± 4 | 31,4 |

Die Ergebnisse zeigen, daß mit Zinn in Form von Dibutylzinndilaurat die Reaktivität der Polyisocyanate gegenüber Polyhydroxylverbindungen für die Herstellung von Blockschäumen eingestellt werden kann. Des weiteren sind die Reaktivität und der Isocyanatgehalt über einen Zeitraum von 8 Monaten konstant, d.h., die Polyisocyanate sind lagerstabil.

In den Vergleichsexperimenten (16v) kann für die Polyisocyanate zwar die gewünschte Reaktivität nachgewiesen werden, doch ist diese nicht über eine Lagerungzeit konstant, d.h. die Polyisocyanate mit Fe-III-chlorid sind nicht lagerstabil.

### ii) Charakterisierung der Rißbildung bei der Herstellung von Formkörpern aus Hartschaumstoffen:

Zur Herstellung der Hartschaumstoffe, anhand derer die Rißbildung untersucht wurde, wurden die folgenden A-Komponenten verwendet:
A1-Komponente, enthaltend:

| | |
|---|---|
| 65,3 Gew.-% | einer Polyhydroxylverbindung, hergestellt aus Saccharose, Glycerin und Propylenoxid, mit einer Hydroxylzahl von 440 mg KOH/g, |
| 13,5 Gew.-% | einer Polyhydroxylverbindung basierend auf einem Amin mit einer Hydroxylzahl von 115 mg KOH/g, |
| 4,5 Gew.-% | einer Polyhydroxylverbindung, hergestellt aus Propylenglykol und Propylenoxid, mit einer Hydroxylzahl von 250 mg KOH/g, |
| 2,24 Gew.-% | des Schaumstabilisators SR 321, Fa. OSI Specialties, |
| 2,58 Gew.-% | eines Katalysatorgemisches aus Aminen, |
| 1,97 Gew.-% | Wasser und |
| 9,91 Gew.-% | Cyclopentan. |

A2-Komponente, enthaltend:

| | |
|---|---|
| 48,9 Gew.-% | einer Polyhydroxylverbindung, hergestellt aus Saccharose, Glycerin und Propylenoxid, mit einer Hydroxylzahl von 445 mg KOH/g, |
| 20,9 Gew.-% | eines reaktiven Flammschutzmittels mit einer Hydroxylzahl von 86 mg KOH/g, |
| 13,0 Gew.-% | Trichlorpropylphosphat, |
| 3,64 Gew.-% | Glycerin, |
| 0,86 Gew.-% | eines Stabilisatorgemisches, |
| 1,68 Gew.-% | Wasser, |
| 3,2 Gew.-% | eines Amin-Katalysatorgemisches, |
| 7,82 Gew.-% | des Treibmittels R 141 b. |

A3-Komponente, enthaltend:

| | |
|---|---|
| 33,0 Gew.-% | einer Polyhydroxylverbindung, hergestellt aus Sorbit, Wasser und Propylenoxid, mit einer Hydroxylzahl von 340 mg KOH/g, |
| 10,7 Gew.-% | Polyethylenglykol mit einer Hydroxylzahl von 190 mg KOH/g, |
| 13,2 Gew.-% | eines Polyesteralkohols hergestellt aus Dimerfettsäure mit einer Hydroxylzahl von 400 mg KOH/g, |
| 30,8 Gew.-% | eines Flammschutzmittels auf der Basis von Chlor, |
| 1,76 Gew.-% | eines Stabilisatorgemisches, |
| 1,23 Gew.-% | Wasser, |
| 1,25 Gew.-% | Kaliumacetat, |
| 1,06 Gew.-% | eines Amin-Katalysatorgemisches und |
| 7,0 Gew.-% | Cyclopentan. |

A4-Komponente, enthaltend:

| | |
|---|---|
| 51,88 Gew.-% | einer Polyhydroxylverbindung, hergestellt aus Phthalsäureanhydrid und Glykolen, mit einer Hydroxylzahl von 240 mg KOH/g, |
| 3,92 Gew.-% | einer Polyhydroxylverbindung, hergestellt aus Sorbit, Wasser und Propylenoxid, mit einer Hydroxylzahl von 490 mg KOH/g, |
| 12,22 Gew.-% | einer Polyhydroxylverbindung, hergestellt aus Propylenglykol, Glycerin und Propylenoxid, mit einer Hydroxylzahl von 400 mg KOH/g, |
| 19,14 Gew.-% | eines Flammschutzmittels auf der Basis von Phosphor und Chlor, |
| 2,55 Gew.-% | eines Stabilisatorgemisches, |
| 1,09 Gew.-% | Wasser, |
| 0,73 Gew.-% | eines Amin-Katalysatorgemisches, |
| 0,82 Gew.-% | Kaliumcarboxylat, |
| 0,36 Gew.-% | eines säurehaltigen Reaktionsverzögerers, |
| 7,29 Gew.-% | n-Pentan. |

Die A-Komponenten und die Polyisocyanate wurden entsprechend der Ausführungen in den Beispielen gemischt. Anschließend wurden 672 g dieses Reaktionsgemisches in ein auf 45 °C temperiertes Formwerkzeug mit den Abmessungen 300 mm × 400 mm × 80 mm gegossen, das danach verschlossen wurde. Nach einer Formstandzeit von 5 bis 10 min wurden die Formkörper 24 h bei Raumtemperatur gelagert, anschließend mittig über die 400 mm Länge zersägt und die Sägeflächen des Formkörpers durch ein Kopiergerät im Maßstab 1 : 1 dokumentiert und auf Rißbildung untersucht. Die Auswertung erfolgte durch Ausmessen der Rißdicken bei 100, 150, 200, 250 und 300 mm Länge des Formkörpers und Mittelwertbildung.

### Beispiel 1

372 g des Polyisocyanates 7e wurden mit der 300 g der A1-Komponente gemischt. Durch einen Vorversuch wurde eine Abbindezeit von 47 s ermittelt. Die Formstandzeit betrug 7 min. Zum Vergleich wurde das Polyisocyanat (a) ohne erfindungsgemäße Zugabe von Katalyasatoren nach der gleichen Vorschrift umgesetzt. Die Abbindezeit wurde durch eine erhöhte Zugabe von Aminkatalysatoren auf ebenfalls 47 s eingestellt. Die Ergebnisse sind in Tabelle 4 aufgeführt.

**Tabelle 4:**

| Polyisocyanat | Reaktionszeit [s] | mittlere Rißdicke [mm] |
|---|---|---|
| 7e | 422 | 1,4 |
| (a) | 886 | 1,8 |

Das erfindungsgemäße Polyisocyanat 7e ist aufgrund der Reaktionszeit sehr gut zur Herstellung von Blockschäumen geeignet. Mit der niedrigen Rißdicke von 1,4 mm eignet sich das Isocyanat aber auch für ein hochkatalysiertes PUR-System sehr gut. Im Gegensatz dazu ist das im Vergleich eingesetzte Polyisocyanat (a) mit einer Reaktionszeit von 886 s nicht zur Herstellung von Blockschäumen geeignet. Zudem wies der mit dem Polyisocyanat (a) hergestellte Hartschaum eine verstärkte Rißbildung auf.

### Beispiel 2

395,5 g des Polyisocyanates 1-3e, 11-15v oder (b) wurden mit 276,5 g der A2-Komponente mit einem Laborrührwerk, das mit einem Rührorgan der Fa. Vollrath ausgestattet war, mit 1750 Umdrehungen pro Minute vermischt und unmittelbar danach in das temperierte Formwerkzeug gegossen. Die Formstandzeit betrug 5 min, wobei die Abbindezeit in den jeweiligen Experimenten analog zu Beispiel 1 mit 46 s konstant gehalten wurde. Dieses Beispiel wurde mit den erfindungsgemäßen Polyisocyanaten 1e bis 3e sowie zum Vergleich mit den Polyisocyanaten (b), 11v bis 15v erarbeitet. Die Ergebnisse sind in Tabelle 5 aufgeführt.

**Tabelle 5:**

| Polyisocyanat | mittlere Rißdicke [mm] |
|---|---|
| 1e | <0,05 |
| 2e | 0,2 |
| 3e | 0,2 |
| (b) | 0,3 |
| 11v | 0,5 |
| 12v | 0,6 |
| 13v | 0,6 |
| 14v | 2,5 |
| 15v | 1,0 |

Für die mit der Polyhydroxylkomponente A2 und den erfindungsgemäßen Polyisocyanaten hergestellten Hartschäumen konnte eine deutlich verringerte Rißbildung festgestellt werden, wobei insbesondere in Falle des Polyisocyanates le die Risse bei einer Dicke von <0,05 mm mit dem bloßen Augen kaum wahrnehmbar waren. Im Vergleich zeigten die Schäume, die mit den Polyisocyanaten (b) sowie v11 bis v15 hergestellt wurden, eine deutlich verstärkte Rißbildung.

### Beispiel 3

372,7 g des Polyisocyanates 8e, 9e, 10e oder (a) wurden mit 299,3 g der A2-Komponente mit einem Laborrührwerk, das mit einem Rührorgan der Fa. Vollrath ausgestattet war, mit 1750 Umdrehungen pro Minute vermischt und unmittelbar danach in das temperierte Formwerkzeug gegossen. Die Formstandzeit betrug 5 min, wobei die Abbindezeit in den jeweiligen Experimenten analog zu Beispiel 1 mit 46 s konstant gehalten wurde. Dieses Beispiel wurde mit den erfindungsgemäßen Polyisocyanaten 8e bis 10e im Vergleich mit dem Polyisocyanat (a) erarbeitet. Die Ergebnisse sind in Tabelle 6 dargestellt.

**Tabelle 6:**

| Polyisocyanat | mittlere Rißdicke [mm] |
|---|---|
| 8e | 21,0 |
| 9e | 12,4 |
| 10e | 14,8 |
| (a) | 33,4 |

Die mit den erfindungsgemäßen Polyisocyanaten hergestellten Hartschäume zeigen im Vergleich zum Polyisocyanat (a) eine deutlich verringerte Tendenz zur Rißbildung. Die im Vergleich zum Beispiel 2 generell verstärkte Tendenz zur Rißbildung ist in den Unterschieden in der Zusammensetzung der Reaktionsgemische zu erklären. In allen Beispielen zeigte sich eine deutlich verminderte Tendenz zur Rißbildung der Hartschäume durch die Herstellung mit den erfindungsgemäßen Polyisocyanaten im Vergleich zu Vergleichsgemischen.

### Beispiel 4 (PUR/PIR-Hartschaum)

448 g des Polyisocyanates 1e, 5e oder (b) wurden mit 224 g der A3-Komponente mit einem Laborrührwerk, das mit einem Rührorgan der Fa. Vollrath ausgestattet war, mit 1750 Umdrehungen pro Minute vermischt und unmittelbar danach in das temperierte Formwerkzeug gegossen. Die Formstandzeit betrug 5 min, wobei die Abbindezeit in den jeweiligen Experimenten analog zu Beispiel 1 mit 47 s konstant gehalten wurde. Dieses Beispiel wurde mit den erfindungsgemäßen Polyisocyanaten 1e bis 5e im Vergleich mit dem Polyisocyanat (b) erarbeitet. Die Ergebnisse sind in Tabelle 7 dargestellt.

**Tabelle 7:**

| Polyisocyanat | mittlere Rißdicke [mm] |
|---|---|
| 1e | 5,4 |
| 5e | 4,8 |
| (b) | 13,4 |

Auch bei der Herstellung der Polyurethan/Polyisocyanurat-Hartschäumen weisen die Hartschäumen, die mit den erfindungsgemäßen Polyisocyanaten hergestellt wurden, eine deutlich verringerte Tendenz zur Rißbildung im Vergleich mit dem Polyisocyanat (b) auf.

### Beispiel 5 (PUR/PIR-Block-Hartschaum)

461,5 g des Polyisocyanates 4e wurden mit 288,5 g der A4-Komponente mit einem Laborrührwerk, das mit einem Rührorgan der Fa. Vollrath ausgestattet war, mit 1750 Umdrehungen pro Minute gemischt. Anschließend wurden 750 g dieser Mischung in einem temperierten Formwerkzeug mit den Abmessungen von 200 mm × 200 mm × 200 mm frei, d.h. ohne Verschließen des Formwerkzeuges, verschäumt. Die Reaktionszeit lag bei 410 s. Der Hartschaum wies eine gleichmäßig feine Zellstruktur ohne Rißbildung auf.

### Beispiel 6

339,0 g des Polyisocyanates 9e, 17-19v oder (b) wurden mit 237,0 g der A2-Komponente mit einem Laborrührwerk, das mit einem Rührorgan der Fa. Vollrath ausgestattet war, mit 1750 Umdrehungen pro Minute vermischt und unmittelbar danach in das auf 45°C temperierte Formwerkzeug mit den Innenabmessungen 400 mm × 300 mm × 80 mm gegossen. Die Formstandzeit betrug 5 min, wobei die Abbindezeit jedesmal analog zu Beispiel 1 mit 46 s konstant gehalten wurde. Wegen der gegenüber den vorangegangenen Beispielen geringeren Dichte des Gesamtformkörpers von 60 kg/m³ ist zwar das Niveau der Rißstärke deutlich vermindert, man erkennt jedoch klar den Einfluß der Zinnverbindung. Mit steigendem Zinngehalt über die erfindungsgemäße Konzentration von 20 ppm Zinn als Zinn-(II)-octoat hinaus erhöht sich die Rißbildung in unerwünschter Weise und außerdem bildet sich eine unbrauchbare, grobzellige Schaumstruktur. Die Ergebnisse sind in Tabelle 8 dargestellt.

**Tabelle 8:**

| Polyisocyanat | mittlere Rißdicke (mm) | Bemerkung |
|---|---|---|
| (b) | 4,5 | |
| 9e | 0,8 | |
| 17v | 1,2 | grobzellige, unbrauchbare Schaumstruktur |
| 18v | 3,4 | grobzellige, unbrauchbare Schaumstruktur |
| 19v | 4,8 | grobzellige, unbrauchbare Schaumstruktur |

### Beispiel 7

Je 50 g Isocyanate 9e, 17v und 19v wurden bei 22°C und 53% rel. Luftfeuchte in 100 ml Bechergläsern 4 Stunden mit offenem Kontakt zur Luft gelagert, um die Resistenz gegen unerwünschte Haut- oder Krustenbildung, d.h. die Lagerfähigkeit, zu beurteilen.

| | |
|---|---|
| Polyisocyanat 9e | keine Haut, keine Kruste am Rand |
| Polyisocyanat 17v | Krustenbildung am Rand |
| Polyisocyanat 19V | Hautbildung |

### Beispiel 8

339,0 g des Polyisocyanates 20e oder (c) wurden mit 237,0 g der A2-Komponente mit einem Laborrührwerk, das mit einem Rührorgan der Fa. Vollrath ausgestattet war, mit 1750 Umdrehungen pro Minute vermischt und unmittelbar danach in das auf 45°C temperierte Formwerkzeug mit den Innenabmessungen 400 mm × 300 mm × 80 mm gegossen. Die Formstandzeit betrug 5 min, wobei die Abbindezeit analog zu Beispiel 1 mit 46 s konstant gehalten wurde.

Die Ergebnisse sind in Tabelle 9 dargestellt.

**Tagelle 9:**

| Polyisocyanat | mittlere Rißdicke [mm] | Reaktionszeit [s] |
|---|---|---|
| 20e | 1,4 | 486 |
| (c) | 1,4 | 716 |

Die Reaktivität der Isocyanatkomponente (c) ist mit 716 s ungeeignet für die Herstellung von Hartschaum-Blockwaren. Im Gegensatz dazu konnte für die erfindungsgemäße Isocyanatkomponente 20e mit 4 ppm Zinn als Di-n-octylzinnmercaptid (Chemiewerk Greiz-Dölau GmbH, Liebigstraße 7, 0-6600 Greiz) eine geeignete Reaktivität (486s) nachgewiesen werden.

Zusammenfassend stellen die Beispiele die folgenden Vorteile der erfindungsgemäßen Polyisocyanate dar:
- Die Reaktivität der Polyisocyanate gegenüber Substanzen, die gegenüber Isocyanaten reaktiv sind, ist hervorragend einstellbar;
- Die Polyisocyanate zeigen eine sehr gute Lagerfähigkeit aufgrund der zeitlich stabilen Reaktivität;
- PUR- oder PUR/PIR-Hartschaumstoffe, insbesondere solche mit einem großen Mindestdurchmesser, die mit den Polyisocyanaten hergestellt wurden, weisen eine deutlich verringerte Rißbildung auf.

## Patentansprüche

1. Verwendung von Polyisocyanaten, enthaltend Zinn in Form von Verbindungen des vier- oder/und zweiwertigen Zinns in einer Menge von 0,1 bis 20 ppm Zinn, bezogen auf die Masse der Polyisocyanate, zur Herstellung von Hartschaumstoffen auf der Basis von Polyurethanen und/oder Polyisocyanuraten mit einer verringerten Rißbildung.

2. Verwendung nach Anspruch 1 von Verbindungen des vier- oder/ und zweiwertigen Zinns der folgenden Formel für das vierwertige Zinn:
RₙSnX₍₄₋ₙ₎
bzw. der folgenden Formel für das zweiwertige Zinn:
RₘSnX₍₂₋ₘ₎
in denen R einen aliphatischen Rest, einen substituierten oder unsubstituierten aromatischen Rest, einen Carbonsäurerest, einen Carbonyl-, Mercaptid- und/oder Thiocarbonylrest mit 1 bis 30 Kohlenstoffen bedeutet, X einen anorganischen Rest darstellt, n 0, 1, 2, 3 oder 4 und m 0, 1 oder 2 bedeuten.

3. Verwendung nach den Ansprüchen 1 und 2 von Zinn in einer Menge von 1 bis 15 ppm, bezogen auf die Masse des Polyisocyanates.

4. Verwendung nach Anspruch 3 von Zinn in einer Menge von 2 bis 10 ppm, bezogen auf die Masse des Polyisocyanates.

5. Verwendung nach den Ansprüchen 1 bis 4 von Dibutylzinndilaurat und/oder Zinn-(II)-octoat und/oder Di-n-octylzinnmercaptid.

6. Verwendung nach den Ansprüchen 1 bis 5 von Mischungen aus 2,4'-, 2,2'- und/oder 4,4'-Diphenylmethan-diisocyanaten und Polyphenylpolymethylen-polyisocyanaten (Roh-MDI).

7. Verfahren zur Herstellung von Hartschaumstoffen mit einer verringerten Rißbildung auf der Basis von Polyurethanen und/ oder Polyisocyanuraten durch Umsetzung von (a) Polyisocyanate mit (b) gegenüber Isocyanaten reaktiven Substanzen und gegebenenfalls (c) Kettenverlängerungs- und/oder Vernetzungsmitteln, (d) Treibmitteln, (e) Katalysatoren und/oder (f) Hilfs- und Zusatzstoffen, dadurch gekennzeichnet, daß das Äquivalenzverhältnis von NCO-Gruppen der Polyisocyanate (a) zur Summe der reaktiven Wasserstoffatome der Komponenten (b) und gegebenenfalls (c) bei Hartschaumstoffen auf Basis von Polyurethanen 0,85 bis 1,80:1 beträgt und bei Hartschaumstoffen auf der Basis von Polyisocyanuraten 1,5 bis 10:1 beträgt und daß man die Umsetzung in Gegenwart von 0,1 bis 20 ppm vier- und/oder zweiwertigem Zinn, bezogen auf die Masse der Polyisocyanate, durchführt, gegenüber Isocyanaten reaktive Verbindungen mit einer Hydroxyzahl von 150 bis 850 mg KOH/g sowie Wasser verwendet und die Polyurethane und/oder Polyisocyanurate einen Mindestdurchmesser von 40 bis 2000 mm aufweisen, wobei sich der der Mindestdurchmesser durch den Durchmesser der größten in die Form der Polyurethane und/oder Polyisocyanurate einschreibbaren Kugel ergibt.

8. Hartschaumstoffe auf der Basis von Polyurethanen und/oder Polyisocyanuraten erhältlich nach einem Verfahren gemäß Anspruch 7.

9. Verwendung der Hartschaumstoffe gemäß Anspruch 8 als Material zur Wärmedämmung in Kühl- und/oder Heizgeräten oder in Sandwichelementen oder in Rohrleitungen.

## Claims

1. Use of polyisocyanates containing tin in the form of compounds of tetravalent or/and divalent tin in an amount of from 0.1 to 20 ppm of tin, based on the mass of the polyisocyanate, for producing rigid foams based on polyurethanes and/or polyisocyanurates and having reduced crack formation.

2. Use as claimed in claim 1 of compounds of tetravalent or/and divalent tin of the following formula for tetravalent tin:
RₙSnX₍₄₋ₙ₎
or the following formula for divalent tin:
RₘSnX₍₂₋ₘ₎
where R is an aliphatic radical, a substituted or unsubstituted aromatic radical, a carboxylic acid radical, a carbonyl, mercaptide and/or thiocarbonyl radical having from 1 to 30 carbon atoms, X is an inorganic radical, n is 0, 1, 2, 3 or 4 and m is 0, 1 or 2.

3. Use as claimed in claim 1 or 2 of tin in an amount of from 1 to 15 ppm, based on the mass of the polyisocyanate.

4. Use as claimed in claim 3 of tin in an amount of from 2 to 10 ppm, based on the mass of the polyisocyanate.

5. Use as claimed in any of claims 1 to 4 of dibutyltin dilaurate and/or tin(II) octoate and/or di-n-octyltin mercaptide.

6. Use as claimed in any of claims 1 to 5 of a mixture of diphenylmethane 2,4'-, 2,2'- and/or 4,4'-diisocyanates and polyphenylpolymethylene polyisocyanates (raw MDI).

7. A process for producing rigid foams having reduced crack formation and based on polyurethanes and/or polyisocyanurates by reacting (a) polyisocyanates with (b) substances reactive toward isocyanates and, if desired, (c) chain extenders and/or crosslinkers, (d) blowing agents, (e) catalysts and/or (f) auxiliaries and additives, wherein the equivalence ratio of NCO groups in the polyisocyanates (a) to the sum of the reactive hydrogen atoms in the components (b) and, if used, (c) is from 0.85 - 1.80:1 in the case of rigid foams based on polyurethanes and is from 1.5 - 10:1 in the case of rigid foams based on polyisocyanurates and the reaction is carried out in the presence of from 0.1 to 20 ppm of tetravalent and/or divalent tin, based on the mass of the polyisocyanates, compounds reactive toward isocyanates and having a hydroxyl number of from 150 to 850 mg KOH/g and also water are used and the polyurethanes and/or polyisocyanurates have a minimum diameter of from 40 to 2000 mm, where the minimum diameter is given by the diameter of the largest sphere which can be inscribed within the shape of the polyurethanes and/or polyisocyanurates.

8. A rigid foam based on polyurethanes and/or polyisocyanurates obtainable by a process as claimed in claim 7.

9. Use of the rigid foam as claimed in claim 8 as material for thermal insulation in cooling and/or heating appliances or in sandwich elements or in pipes.

## Revendications

1. Utilisation de polyisocyanates contenant de l'étain sous forme de composés de l'étain tétravalent et/ou bivalent en une quantité de 0,1 à 20 ppm d'étain, rapportées à la masse des polyisocyanates, pour préparer des mousses dures à base de polyuréthanes et/ou de polyisocyanurates manifestant une fissuration réduite.

2. Utilisation selon la revendication 1 de composés de l'étain tétravalent et/ou bivalent répondant à la formule ci-après pour l'étain tétravalent:
RₙSnX₍₄₋ₙ₎
respectivement à la formule ci-après pour l'étain bivalent:
RₘSnX₍₂₋ₘ₎
dans lesquelles R représente un radical aliphatique, un radical aromatique substitué ou non substitué, un radical d'acide carboxylique, un radical carbonyle, un radical mercaptide et/ou un radical thiocarbonyle contenant de 1 à 30 atomes de carbone, X représente un radical inorganique, n représente 0, 1, 2 , 3 ou 4 et m représente 0, 1 ou 2.

3. Utilisation d'étain selon les revendications 1 et 2, en une quantité de 1 à 15 ppm rapportées à la masse du polyisocyanate.

4. Utilisation d'étain selon la revendication 3, en une quantité de 2 à 10 ppm rapportées à la masse du polyisocyanate.

5. Utilisation, selon les revendications 1 à 4, de dilaurate de dibutylétain et/ou d'octoate d'étain (II) et/ou de mercaptide de di-n-octylétain.

6. Utilisation, selon les revendications 1 à 5, de mélanges du 2,4'-, du 2-2' et/ou du 4,4'-diphénylméthane-diisocyanates et de polyphénylpolyméthylène-polyisocyanates (MDI brut).

7. Procédé pour la préparation de mousses dures à fissuration réduite à base de polyuréthanes et/ou de polyisocyanurates par mise en réaction (a) de polyisocyanates avec (b) des substances réactives vis-à-vis d'isocyanates et, le cas échéant, (c) d'agents d'allongement de chaînes et/ou de réticulation, (d) d'agents moussants, (e) de catalyseurs et/ou (f) d'adjuvants et d'additifs, caractérisé en ce que le rapport d'équivalence des groupes NCO des polyisocyanates (a) à la somme des atomes d'hydrogène réactifs du composant (b) et, le cas échéant, du composant (c) dans des mousses dures à base de polyuréthanes s'élève de 0,85 à 1,80:1 et dans des mousses dures à base de polyisocyanurates de 1,5 à 10:1 et en ce qu'on effectue la mise en réaction en présence de 0,1 à 20 ppm d'étain tétravalent et/ou bivalent rapportées à la masse des polyisocyanates; on utilise des composés réactifs vis-à-vis d'isocyanates possédant un indice d'hydroxyle de 150 à 850 mg de KOH/g, ainsi que de l'eau; et les polyuréthanes et/ou les polyisocyanurates présentent un diamètre minimal de 40 à 2000 mm, le diamètre minimal étant fourni par le diamètre de la sphère maximale qui peut être inscrite dans la forme des polyuréthanes ou des polycyanurates.

8. Mousses dures à base de polyuréthanes et/ou de polyisocyanurates, que l'on obtient conformément à un procédé selon la revendication 7.

9. Utilisation des mousses dures selon la revendication 8 à titre de matériaux pour l'isolation thermique dans des appareils de refroidissement et/ou de chauffage ou encore dans des éléments en sandwich ou dans des conduits tubulaires.
